# EUROPEAN PATENT SPECIFICATION

(11) **EP 1 592 410 B1**
(45) Date of publication and mention of the grant of the patent: **23.07.2008**
(21) Application number: 04710282.7
(22) Date of filing: 11.02.2004
(51) Int. Cl.: A61K 9/22, A61K 9/24

(54) **METHODS AND DOSAGE FORMS WITH MODIFIED LAYER GEOMETRY**
VERFAHREN UND DOSIERFORMEN MIT MODIFIZIERTER SCHICHTGEOMETRIE
METHODES ET FORMES DOSIFIEES PRESENTANT UNE GEOMETRIE DE COUCHE MODIFIEE

(30) Priority: 11.02.2003 US 446425 P
(43) Date of publication of application: 09.11.2005
(73) Proprietor: Alza Corporation, Mountain View, CA 94039-7210 (US)
(72) Inventor: SUBRAMANIAN, Ramkumar, Sunnyvale, CA 94085 (US); HEARNEY PORS, Linda, Saratoga, CA 95070 (US); REN, Feiyan, Cupertino, CA 95014 (US); HAN, Jasmine, Emeryville, CA 94608 (US); BARCLAY, Brian, L., Sunnyvale, CA 94087 (US)
(74) Representative: Peebles, Katrina
(86) International application number: PCT/US2004/004299
(87) International publication number: WO 2004/071497

(56) References cited:
- US-A- 4 111 202
- US-A- 4 717 566
- US-A- 5 232 705
- US-A1- 2002 086 055
- US-A1- 2002 146 453

## Description

### FIELD OF THE INVENTION

This invention pertains to the controlled delivery of pharmaceutical agents via dosage forms. In particular, the invention is directed to dosage forms for the controlled delivery of cyclobenzaprine hydrochloride. More particularly, the present application describes methods of enhancing release rates by modifying the geometry of core layers to alter the mixing of layers during operation in addition to modifying the composition of core layers to alter the viscosity during operation.

### BACKGROUND OF THE INVENTION

Traditional osmotic delivery devices control the rate of release from the dosage form through adjustments in the composition of the outer membrane in order to control the osmosis of fluid into the dosage form. The present invention takes advantage of the diffusional and viscous properties of the core.layers in multilayer dosage forms to further define a particular delivery pattern. Manipulating the geometry of compression and the composition of the core layers to after the relative viscosity of the hydrated layers during operation generates enhanced release profiles.

The art is replete with descriptions of oral dosage forms for the controlled release of pharmaceutical agents.
US 2002/0086055 addresses problems associated with the delivery of liquid drugs. The dosage forms disclosed in this document attempt to solve this problem by absorbing the liquid drug into porous particles without significant exudation of the liquid drug from the porous particles. The document discloses a dosage form comprising (a) a membrane defining a compartment, the membrane having an exit orifice and being semipermeable; (b) an expandable layer located remote from the exit orifice; (c) a delay layer located adjacent the exit orifice; and (d) a drug layer located between the delay and the expandable layer.
While a variety of sustained release dosage forms for delivering certain drugs exhibiting short half-life may be known, not every drug may be suitably delivered from those dosage forms because of solubility, metabolic processes, absorption and other physical, chemical and physiological parameters that may be unique to the drug and the mode of delivery. Examples of such drugs that are not likely candidates for controlled release dosage forms are those exhibiting a long half-life such as the tricyclic amine cyclobenzaprine hydrochloride.

Similarly, cyclobenzaprine is relatively highly soluble in aqueous solutions providing greater difficulty in controlling it release from a dosage form over a prolonged period of time at a uniform rate of release. This is evident in the present invention where an initial bolus delivery is followed by a desired delay in delivery of active agent followed by an ascending rate of release of active agent at a uniform rate over a prolonged period of time. The high solubility of the active agent has provided difficulty in controlling for a desired delay.

Cyclobenzaprine hydrochloride is indicated for treatment of muscle spasms associated with acute, painful muscoloskelatal conditions. It is freely soluble in water and alcohol, sparingly soluble in isopropanol, and insoluble in hydrocarbon solvents. Additionally, since cyclobenzaprine has a significantly long half-life (1-3 days) Physicians' Desk Reference, Thompson Healthcare, 56th Ed., pp. 572-573 (2002), and it is not a typical candidate for extended delivery. However, side effects such as drowsiness and dry mouth appear to be related to high blood plasma concentration levels restricting the ability to administer a single daily immediate release dose.

Cyclobenzaprine, like other tricyclic amine salts such as amitriptyline, has reported side effects of sedation and dry mouth. It is expected that the side effects are likely a result of either rate of rise and/or actual drug blood plasma concentrations exceeding a threshold maximum tolerable concentration (MTC). However, in order to obtain a therapeutic effect, concentrations need to be sustained above a minimum pharmacodynamic concentration (MPC).

Another aspect of delivery of cyclobenzaprine is that administration often requires high drug loading in the dosage form. Dosage forms may need to contain drug in the range of 20% to 90% of the overall weight of the dosage form. Such high drug loading requirements present problems in formulating compositions and fabricating dosage forms that are suitable for oral administration and can be swallowed without undue difficulty. Additionally, such loading requirements may present problems when formulating dosage forms that are administered a limited number of times per day, such as for once-a-day dosing, with a goal of release of active agent over a prolonged period of time.

Exemplary controlled release dosage forms include, US Pat. No. 5,536,507 which describes a three component pharmaceutical formulation that utilizes, *inter alia*, a pH sensitive polymer and optionally an osmotic agent that will swell in the higher pH regions of the lower portion of the small intestine and the large intestine to release drug in those environments. Additional components of the dosage form include a delayed release coating and an enteric coating to provide a dosage form that releases very little, if any, of the drug in the stomach, a relatively minimal amount in the small intestine and reportedly about 85% or more in the large intestine. Such a dosage form provides for a widely varying time-release of drug after administration that may not begin for 1-3 hours until the dosage form has passed from the stomach and an additional 3 hours or more for the dosage form to pass into the large intestine.

Exemplary sustained release cyclobenzaprine dosage forms, methods of preparing such dosage forms and methods of using such dosage forms are described herein directed to osmotic dosage forms for oral administration.

In addition to osmotic systems as described herein, however, there are many other approaches to achieving sustained release of drugs from oral dosage forms known in the art. These different approaches include, for example, diffusion systems such as reservoir devices and matrix devices, dissolution systems such as encapsulated dissolution systems (including, for example, "tiny time pills") and matrix dissolution systems, combination diffusion/dissolution systems and ion-exchange resin systems as described In Remington's Pharmaceutical Sciences, 1990 ed., pp. 1682-1685.

Osmotic dosage forms in general utilize osmotic pressure to generate a driving force for imbibing fluid into a compartment formed, at least in part, by a semipermeable membrane that permits free diffusion of fluid but not drug or osmotic agent(s), if present A significant advantage to osmotic systems is that operation is pH-independent and thus continues at the osmotically determined rate throughout an extended time period even as the dosage form transits the gastrointestinal tract and encounters differing microenvironments having significantly different pH values. A review of such dosage forms is found in Santus and Baker, "Osmotic drug delivery: a review of the patent literature," Journal of Controlled Release 35 (1995) 1-21.
In particular, the following U.S. Patents, owned by the assignee of the present application, ALZA Corporation, directed to osmotic dosage forms: Nos. 3,845,770; 3,916,899; 3,995,631; 4,008,799; 4,111,202; 4,160,020; 4,327,725; 4,519,801; 4,578,075; 4,681,583; 5,099,397; and 5,156,850.

Devices in which a drug composition is delivered as a slurry, suspension or solution from a small exit orifice by the action of an expandable layer are described in U. S. Patents Nos. 5,633,011; 5,190,765; 5,252,338; 5,620,705; 4,931,285; 5,006,346; 5,024,842; and 5,160,743. Typical devices include an expandable push layer and a drug layer surrounded by a semipermeable membrane. In certain instances, the drug layer is provided with a subcoat to delay release of the drug composition to the environment of use or to form an annealed coating in conjunction with the semipermeable membrane.

Devices in which a drug composition is delivered in a dry state from a large exit orifice by the action of an expandable layer are described in US Patent Nos. 4,892,778, 4,915,949 and 4,940,465. Those references describe a dispenser for delivering a beneficial agent to an environment of use that includes a semipermeable wall containing a layer of expandable material that pushes a dry drug layer out of the compartment formed by the wall. The exit orifice in the device is substantially the same diameter as the inner diameter of the compartment formed by the wall.

While dosage forms delivering the drug composition to the environment of use in the dry state may provide suitable release of drug at high drug loading over a prolonged period of time, the exposure of the drug layer to the environment of use may result in agitation-dependent release of drug that in some circumstances is difficult to control. Accordingly, it may be advantageous to release the drug as a slurry or suspension that may be metered by control of rate of expansion of the push layer and the size of the exit orifice in the dosage form as in accordance with this invention.

US Patent 5,169,638 describes a buoyant controlled release pharmaceutical powder formulation to be filled into capsules that uses a pH dependent polymer formed from alginic acid and hydroxypropylmethyl cellulose to release pharmaceuticals at a controlled rate. It appears that this capsule formulation was intended to mimic the characteristics of a tableted formulation. No description is provided of a formulation that provides the uniform release characteristics of the dosage forms containing cyclobenzaprine and related compounds of the present invention.

US Patent Nos. 4,892,778 and 4,940,465, describe a dispenser for delivering a beneficial agent to an environment of use that includes a semipermeable wall containing a layer of expandable material that pushes a drug layer out of the compartment formed by the wall. The exit orifice in the device is substantially the same diameter as the inner diameter of the compartment formed by the wall.

US Patent No. 4,915,949, describes a dispenser for delivering a beneficial agent to an environment of use that includes a semipermeable wall containing a layer of expandable material that pushes a drug layer out of the compartment formed by the wall. The drug layer contains discrete tiny pills dispersed in a carrier. The exit orifice in the device is substantially the same diameter as the inner diameter of the compartment formed by the wall.

US Patent No. 5,126,142, describes a device for delivering an ionophore to livestock that includes a semipermeable housing in which a composition containing the ionophore and a carrier and an expandable hydrophilic layer is located, along with an additional element that imparts sufficient density to the device to retain it in the rumen-reticular sac of a ruminant animal. The ionophore and carrier are present in a dry state during storage and the composition changes to a dispensable, fluid-like state when it is in contact with the fluid environment of use. A number of different exit arrangements are described, including a plurality of holes in the end of the device and a single exit of varying diameter to control the amount of drug released per unit time due to diffusion and osmotic pumping.

The prior art is notably silent on taking advantage of the diffusional and relative viscosity properties of the core layers. Prior art osmotic systems while providing advantageous controlled delivery over a prolonged period of time, suffered from periods of inconsistent delivery resulting in bumps, increases or decreases, in the release rate profile. Such bumps can adversely impact blood plasma concentrations. The present invention is able to modify the release rate to provide for more uniform delivery, reducing these bumps. This is achieved by modifying the geometry of core layers to alter the mixing of layers during operation in addition to modifying the composition of core layers to alter the relative viscosity of the hydrated layers during operation.

Although, as described above, there are a variety of sustained release dosage forms for delivering certain drugs, not every drug may be suitably delivered from those dosage forms because of solubility, metabolic processes, absorption and other physical, chemical and physiological parameters that may be unique to the drug and the mode of delivery.

There remains a need for effective dosing methods, dosage forms and devices that will permit the controlled release of the aforementioned compound over a prolonged period of time while reducing undesirable increases or decreases in release rate during operation.

### SUMMARY OF THE INVENTION

According to the present invention, there is provided a dosage form comprising:
(a) a membrane defining a compartment, the membrane having an exit orifice formed or formable therein and at least a portion of the membrane being semipermeable;
(b) an expandable layer located within the compartment remote from the exit orifice and in fluid communication with the semipermeable portion of the membrane;
(c) a delay layer located adjacent the exit orifice;
(d) a drug layer located within the compartment between the delay layer and the expandable layer; and
(e) an interface boundary between the delay layer and the drug layer, the interface boundary being convex in shape relative to the exit orifice.

The present invention is a dosage form designated for once-a-day administration to deliver cyclobenzaprine HCI for approximately 20 hours utilizing a capsule-shaped tablet with an optional drug overcoat. This approximately 20 hours of release is comprised of the immediate release drug overcoat delivery followed by no further drug delivery for about four hours after administration until the core releases drug in a controlled fashion for about 16 hours. This novel profile provides therapeutic delivery while the delay in drug delivery keeps the plasma levels low enough such that side effects are reduced and the development of tolerance is increased. This delivery profile provides 24 hours of efficacy without high plasma levels.

The present invention capitalizes upon diffusional and viscous properties of the core layers to develop greater uniformity in the drug release rate and a more predictable delay period marked by greater demarcation of the end of the delay period and beginning of drug delivery from the core. By modifying the compression sequence to invert the layer geometry of the multilayer dosage form, inter-layer mixing is more uniform. Where the layer geometry is inverted over traditional compaction geometry an enhanced delivery profile of more continuous rate of drug delivery is achieved. In particular, the optimal layer geometry is found when the each layer is convex relative to the orifice, rather than being concave relative to the orifice. Prior multilayer dosage forms relied upon a concave geometry relative to the delivery orifice as a result of the traditional compression sequence.

Additionally, by controlling the delay layer and drug layer formulations such that the delay layer viscosity remains higher than the drug layer viscosity during the desired delay period, premature tunneling of the drug layer through the delay layer can be reduced in order to provide the optimal drug-free release period followed by a continuous and substantially ascending release from the core. Premature tunneling allows the drug layer to be prematurely delivered from the system resulting in fluctuations in the release rate during the ascending release rate period.

Prior multilayer formulations are characterized by having a first layer viscosity during operation of less than the second layer viscosity.

The present invention provides for a modulated release rate that is neither ascending, flat or delayed, but rather a hybrid. It has been surprisingly discovered that the instant profile should provide efficacious therapy over 24 hours while reducing negative side effects associated with administration of the drug. Additionally, while cyclobenzaprine is exemplified herein, the present invention would be equally beneficial to delivery of other drugs in the tricyclic amine antidepressant class including amitriptyline, imipramine and desipramine.

The present invention utilizes a rapid initial bolus delivery where the drug is delivered in the upper gastrointestinal (GI) tract, followed by a delay in delivery of drug for about four hours followed by a slow, but substantially ascending, release when the dosage form is likely to be in the colonic region of the GI tract. This profile was not previously used to deliver any drug, especially cyclobenzaprine and is designed to reduce the impairment of a subject's cognitive functions.

It has been surprisingly found that the described modulated release rate provides for a substantially ascending blood plasma concentration of drug with peak concentration occurring at about 20 hours after administration. This ascending blood plasma concentration should reduce the intraday tolerance effect developed.

The dosage form utilizes a semipermeable membrane surrounding a three-layer core: the first layer is referred to as a delay layer and contains no drug; the second layer referred to as the drug layer contains drug such as cyclobenzaprine HCI and excipients; and the third layer referred to as the push layer contains osmotic agents and no drug. An orifice is drilled through the membrane on the delay-layer end of the capsule-shaped tablet. An exterior drug overcoat is optionally applied to the membrane.

In the aqueous environment of the gastrointestinal (GI) tract, the drug overcoat dissolves. Then, water is imbibed through the semipermeable membrane at a controlled rate determined by the properties of the membrane and the osmolality of the core consitituents. This causes the push layer to swell and the delay and drug layers to hydrate and form viscous, but deformable, masses. The push layer expands against the drug layer, which in turn pushes against the hydrated delay layer. Preferably, the delay layer, followed by the drug layer, exits the system through the orifice in the membrane at the same rate that water is imbibed into the core. In traditional systems, the drug layer may tunnel through the delay layer or the layers may mix in a non-uniform manner and alter the desired release rate profile. The biologically inert components of the tablet remain intact during the GI transit and are eliminated as a shell along with insoluble core components.

As the active drug is relatively highly soluble in an aqueous environment, the drug layer has a tendency to mix into the delay layer. The compression sequence of the present invention is inverted resulting in the geometry of the drug layer and delay layer interface being convex relative to the orifice rather than traditional concave in shape.

Additionally, depending upon the relative viscosity of the drug and delay layers, different release profiles are obtained. It is imperative to identify the optimum viscosity for each layer. It has been found that the optimum viscosity of the layers should be between 70 cps and 350 cps and preferably between about 84 cps and 158 cps and more preferably about 109 cps.

In order to maintain the delay period of the present preferred delivery profile with no drug delivery for the desired duration, mixing is to be reduced and controlled. As such, it has been discovered that it is preferable to create a dosage form wherein the viscosity of the hydrated delay layer remains higher than the viscosity of the hydrated drug layer throughout the delay period. Such a relative viscosity deters premature mixing and tunneling by the drug layer into the delay layer and provides a sharper demarcation between the end of the delay period and the beginning of the ascending release period as well as providing a more uniform delivery from the dosage form during the ascending release period.

The delivery profile from the core is also dependent upon the weight and thickness of each of the core layers. Increasing the thickness or weight of a particular layer increases the delivery time for that portion of the profile. For example, a thicker delay layer provides a longer duration of delay before delivery of active agent from the core commences.

The ratio of core diameter to core length is also an important factor. For example, a narrower core shape provides a shorter period of mixing of the adjacent layers thus a steeper ascending rate, from low release rate to higher release rate of active agent, while a wider core diameter provides a larger interface area for mixing between adjacent layers providing a shallower ascending rate. The shape of the system as a capsule shaped tablet is an important feature that is responsible for providing the somewhat ascending profile from the core. Thus, the shape can vary between a capsule shaped tablet to a standard biconvex shape, with a concomitant change in delivery profile.

The delivery system is designed to achieve plasma concentrations of approximately 6 to 8 ng/ml, and preferably between 6.5 ng/ml and 6.9 ng/ml, three to four hours after dosing and approximately 8 to 12 ng/ml, and preferably between 9.7 ng/ml and 10.2 ng/ml, eighteen to twenty hours after administration of a single 22.5mg dosage form. Peak concentrations occur at approximately twenty hours. Other doses are expected to result in approximately linear fluctuations, higher or lower, in blood plasma concentrations.

The present invention is designed to be a once-a-day dosage form that is therapeutically effective while producing fewer side effects than immediate release dosage forms presently administered multiple times per day. The present invention provides two key features: a modulated delivery that affects the pharmacodynamics and development of tolerance, and the modulated delivery provides adequate blood plasma concentrations for therapeutic effect. The modulated delivery allows a sustained concentration profile above the MPC but does not exceed the MTC. Development of tolerance is related to the sedation effects (an impairment of the cognitive function as measured by a representative measure such as digit vigilance).

The invention comprises layer interface geometry such that each layer interface is convex relative to the orifice. This result is obtained by inverting the traditional compression sequence during manufacturing.

In preferred embodiments, the invention comprises layer compositions to control the viscosity of the layers during operation to enhance the demarcation of the delay and release periods. In particular, the hydrated viscosity of the delay layer is preferably higher than the drug layer during operation.

Preferably, the dosage form is
adapted to release over a prolonged period of time at a modulated rate of release, the compound cyclobenzaprine.

Preferably, the dosage form comprises a means of maintaining a uniform release rate after a delay period of no drug delivery from the dosage form by controlling the viscosity of the hydrated drug layer and the hydrated delay layer.

Also described herein is a method of treating a condition in a subject responsive to administration of cyclobenzaprine or a pharmaceutically acceptable acid addition salt thereof, which comprises orally administering to the subject a dosage form adapted to release the compound at a modulated rate of release over a prolonged period of time. Preferably, the compound is a tricyclic amine and more preferably, cyclobenzaprine, and the dosage form comprises an osmotic material. Most preferably, the dosage form is administered orally, once a day.

The dosage form of the present invention may optionally comprise a flow-promoting layer between the membrane and the drug layer and delay layer.

Also described herein is a method of treating a condition responsive to administration of cyclobenzaprine or a pharmaceutically acceptable acid addition salt thereof, which comprises administering the compound to provide a modulated, substantially ascending, plasma concentration of the compound between 7ng/ml and 11 ng/ml for 16 to 18 hours in the 24 hour administration period for a 20 mg dose. During the 24 hour period after administration of the dosage form the quotient formed by [Cₘₐₓ - Cₘᵢₙ]/Cₘᵢₙ is less than 1. The Cₘₐₓ occurs at a time greater than about 16 hours and preferably at about 20 hours.

### BRIEF DESCRIPTION OF THE FIGURES

Figure 1 illustrates a two-orifice capsule shaped tablet prior to administration to a subject, illustrating an inner lubricating wall with optional external drug overcoat and clear coat;

Figure 2 illustrates a standard biconvex shaped tablet prior to administration to a subject, illustrating an optional inner lubricating wall;

Figure 3 illustrates a single orifice capsule shaped tablet prior to administration to a subject, employing an optional inner lubricating wall and barrier layer,

Figure 4 illustrates the model release rate profile utilizing the optional drug overcoat and ascending delivery rate for a 22.5 mg cyclobenzaprine system;

Figure 5 illustrates the predicted blood plasma concentration time profile resulting from the model delivery profile. Figure 5 also shows the predicted blood plasma concentration profiles for 5 and 10 mg immediate release dosage forms administered three times per day (tid). The dosage form maintains blood plasma concentrations generally comparable to the peak concentrations from 5 mg tid, but below the trough concentration from 10 g tid.

Figure 6 illustrates colonic absorption of cyclobenzaprine, a prerequisite for the present invention.

Figure 7 illustrates sedative effects of the present invention by demonstrating that in spite of higher AUC values on Day 2, the speed of detections AUC is lower compared to Day 1 suggesting the development of tolerance.

Figure 8 illustrates a release profile (release rate as a function of time) of the active agent cyclobenzaprine from a representative dosage form having the general characteristics illustrated in Figure 1, but without the drug overcoat for a range of core viscosities;

Figure 9 illustrates the cumulative release of cyclobenzaprine over time of the active agent cyclobenzaprine from a representative dosage form having the general characteristics illustrated in Figure 1, but without the drug overcoat for a range of core viscosities;

Figure 10 illustrates the release rate profile (release rate as a function of time) of the active agent cyclobenzaprine from a representative dosage form having the general characteristics illustrated in Figure 1, with a drug overcoat and a core viscosity of 109cps;

Figure 11 illustrates the cumulative release of cyclobenzaprine over time of the active agent cyclobenzaprine from a representative dosage form having the general characteristics illustrated in Figure 1, with a drug overcoat and a core viscosity of 109cps;

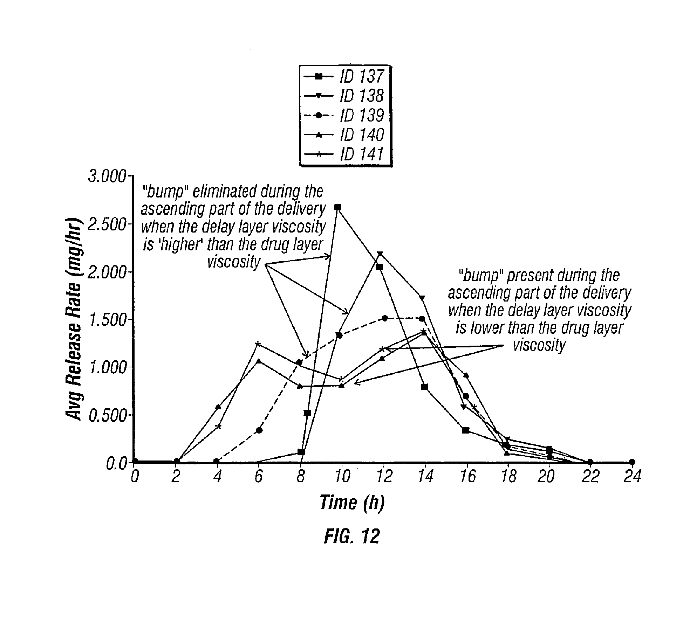

Figure 12 illustrates average release rates when the delay layer viscosity is higher and lower than the drug layer viscosity during operation.

Figure 13 Illustrates the layer interface geometry of the present invention, convex relative to the exit orifice.

Figure 14 illustrates the layer interface geometry of the traditional compression process, concave relative to the exit orifice,

Figure 15 illustrates the effect of the inverted compression sequence on release rate verses the traditional compression sequence for a 15/64" system.

Figure 16 illustrates the effect of the inverted compression sequence on release rate verses the traditional compression sequence for a 13/64" system.

### DETAILED DESCRIPTION OF THE INVENTION

The present invention is best understood by reference to the following definitions, the drawings and exemplary disclosure provided herein.

### DEFINITIONS

By "dosage form" is meant a pharmaceutical composition or device comprising a pharmaceutically active agent, such as cyclobenzaprine or a pharmaceutically acceptable acid addition salt thereof, the composition or device optionally containing inactive ingredients, i.e., pharmaceutically acceptable excipients such as suspending agents, surfactants, disintegrants, binders, diluents, lubricants, stabilizers, antioxidants, osmotic agents, colorants, plasticizers, coatings and the like, that are used to manufacture and deliver active pharmaceutical agents.

By "active agent", "drug", or "compound" is meant an agent, drug, or compound having the characteristics of cyclobenzaprine or a pharmaceutically acceptable acid addition salt thereof.

By "pharmaceutically-acceptable acid addition salt" or "pharmaceutically acceptable salt", which are used interchangeably herein, are meant those salts in which the anion does not contribute significantly to the toxicity or pharmacological activity of the salt, and, as such, they are the pharmacological equivalents of the bases of the cyclobenzaprine compound. Included as well are other tricyclic amine antidepressants of which cyclobenzaprine is representative. Examples of pharmaceutically acceptable acids that are useful for the purposes of salt formation include but are not limited to hydrochloric, hydrobromic, hydroiodic, citric, acetic, benzoic, mandelic, phosphoric, nitric, mucic, isethionic, palmitic, and others.

By "sustained release " is meant a predetermined continuous release of active agent to an environment over a prolonged period.

The expressions "exit," "exit orifice," "delivery orifice" or "drug delivery orifice," and other similar expressions, as may be used herein include a member selected from the group consisting of a passageway; an aperture; an orifice; and a bore. The expression also includes an orifice that is formed or formable from a substance or polymer that erodes, dissolves or is leached from the outer wall to thereby form an exit orifice.

A drug "release rate" refers to the quantity of drug released from a dosage form per unit time, e.g., milligrams of drug released per hour (mg/hr). Drug release rates for drug dosage forms are typically measured as an *in vitro* rate of dissolution, i.e., a quantity of drug released from the dosage form per unit time measured under appropriate conditions and in a suitable fluid. The dissolution tests described herein were performed on dosage forms placed in metal coil sample holders attached to a USP Type VII bath indexer in a constant temperature water bath at 37°C. Aliquots of the release rate solutions were injected into a chromatographic system to quantify the amounts of drug released during the testing intervals.

By "release rate assay" is meant a standardized assay for the determination of the release rate of a compound from the dosage form tested using a USP Type VII interval release apparatus. It is understood that reagents of equivalent grade may be substituted in the assay in accordance with generally accepted procedures.

For clarity and convenience herein, the convention is utilized of designating the time of drug administration as zero hours (t = 0 hours) and times following administration in appropriate time units, e.g., t = 30 minutes or t = 2 hours, etc.

As used herein, unless otherwise specified, a drug release rate obtained at a specified time "following administration" refers to the *in vitro* drug release rate obtained at the specified time following implementation of an appropriate dissolution test. The time at which a specified percentage of the drug within a dosage form has been released may be referenced as the "Tₓ" value, where "x" is the percent of drug that has been released. For example, a commonly used reference measurement for evaluating drug release from dosage forms is the time at which 70% or 90% of drug within the dosage form has been released. This measurement is referred to as the "T₇₀" or "T₉₀" for the dosage form.

By "immediate-release dosage form" is meant a dosage form that releases drug substantially completely within a short time period following administration, i.e., generally within a few minutes to about 1 hour.

By "extended release dosage form" or "controlled release dosage form" is meant a dosage form that releases drug in a substantially consistent predetermined rate for many hours. Controlled release dosage forms in accord with the present invention exhibit T₉₀ values of at least about 16 hours or more and preferably about 18 hours or more. The dosage forms release drug over periods of time of at least about 10 hours, preferably 12 hours or more and, more preferably, 16-20 hours or more.

By "sustained release dosage form" is meant a dosage form that releases drug substantially continuously for many hours. Sustained release dosage forms of the present invention release drug over periods of time of at least about 10 hours, preferably about 12 hours or more and, more preferably, about 16 hours or more.

Dosage forms in accord with the present invention exhibit controlled release rates of cyclobenzaprine for a prolonged period of time.

By "uniform release rate" is meant an average hourly release rate from the core that varies positively or negatively by no more than about 30% and preferably no more than about 25%, most preferably no more than about 10%, from either the preceding or the subsequent average hourly release rate as determined in a USP Type VII Interval Release Apparatus where the cumulative release is between 25% and 75%.

By "prolonged period of time" is meant a continuous period of time of at least about 4 hours, preferably 6-8 hours or more and, more preferably, 10 hours or more. For example, the exemplary osmotic dosage forms described herein generally begin releasing cyclobenzaprine at a uniform release rate at about 4 hours following administration and the uniform rate of release, as defined above, continues for a prolonged period of time from about 25% to until at least about 75% and preferably at least about 85% of the drug is released from the dosage form. Release of cyclobenzaprine continues thereafter for several more hours although the rate of release is generally slowed somewhat from the uniform release rate.

By "C" is meant the concentration of drug in the blood plasma of a subject, generally expressed as mass per unit volume, typically nanograms per milliliter. For convenience, this concentration may be referred to as "plasma drug concentration" or "plasma concentration" herein which is intended to be inclusive of drug concentration measured in any appropriate body fluid or tissue. The plasma drug concentration at any time following drug administration is referenced as Cₜᵢₘₑ, as in C₉ₕ or C₂₄ₕ, etc.

By "steady state" is meant the condition in which the amount of drug present in the blood plasma of a subject does not vary significantly over a prolonged period of time. A pattern of drug accumulation following continuous administration of a constant dose and dosage form at constant dosing intervals eventually achieves a "steady-state" where the plasma concentration peaks and plasma concentration troughs are essentially identical within each dosing interval. As used herein, the steady-state maximal (peak) plasma drug concentration is referenced as Cₘₐₓ and the minimal (trough) plasma drug concentration is referenced as Cₘᵢₙ. The times following drug administration at which the steady-state peak plasma and trough drug concentrations occur are referenced as the Tₘₐₓ and the Tₘᵢₙ, respectively.

Persons of skill in the art appreciate that plasma drug concentrations obtained in individual subjects will vary due to inter-patient variability in the many parameters affecting drug absorption, distribution, metabolism and excretion. For this reason, unless otherwise indicated, mean values obtained from groups of subjects are used herein for purposes of comparing plasma drug concentration data and for analyzing relationships between *in vitro* dosage form dissolution rates and *in vivo* plasma drug concentrations.

It has been surprisingly discovered that sustained release cyclobenzaprine dosage forms exhibiting T₉₀ values of 13-15 hours or more and more preferably 16-18 hours or more and which release cyclobenzaprine at a controlled release rate for a prolonged period of time can be prepared. Administration of such dosage forms once daily provides therapeutically effective average steady-state plasma cyclobenzaprine concentrations.

The exemplary sustained release cyclobenzaprine dosage forms, methods of preparing such dosage forms and methods of using such dosage forms described herein are directed to osmotic dosage forms for oral administration. In addition to osmotic systems as described herein, however, there are many other approaches to achieving sustained release of drugs from oral dosage forms known in the art. These different approaches may include, for example, diffusion systems such as reservoir devices and matrix devices, dissolution systems such as encapsulated dissolution systems (including, for example, "tiny time pills") and matrix dissolution systems, combination diffusion/dissolution systems and ion-exchange resin systems as described in Remington's Pharmaceutical Sciences, 1990 ed., pp. 1682-1685.

Osmotic dosage forms, in general, utilize osmotic pressure to generate a driving force for imbibing fluid into a compartment formed, at least in part, by a semipermeable wall that permits free diffusion of fluid but not drug or osmotic agent(s), if present. A significant advantage to osmotic systems is that operation is pH-independent and thus continues at the osmotically determined rate throughout an extended time period even as the dosage form transits the gastrointestinal tract and encounters differing, microenvironments having significantly different pH values. A review of such dosage forms is found in Santus and Baker, "Osmotic drug delivery: a review of the patent literature," Journal of Controlled Release 35 (1995) 1-21. In particular, the following U.S. Patents, owned by the assignee of the present application, ALZA Corporation, directed to osmotic dosage forms: Nos. 3,845,770; 3,916,899; 3,995,631; 4,008,719; 4,111,202; 4,160,020; 4,327,725; 4,519,801; 4,578,075; 4,681,583; 5,019,397; and 5,156,850.

The present invention improves the uniform release rate of a system having multiple layers by controlling the premature mixing and tunneling of subsequent layers into prior layers.

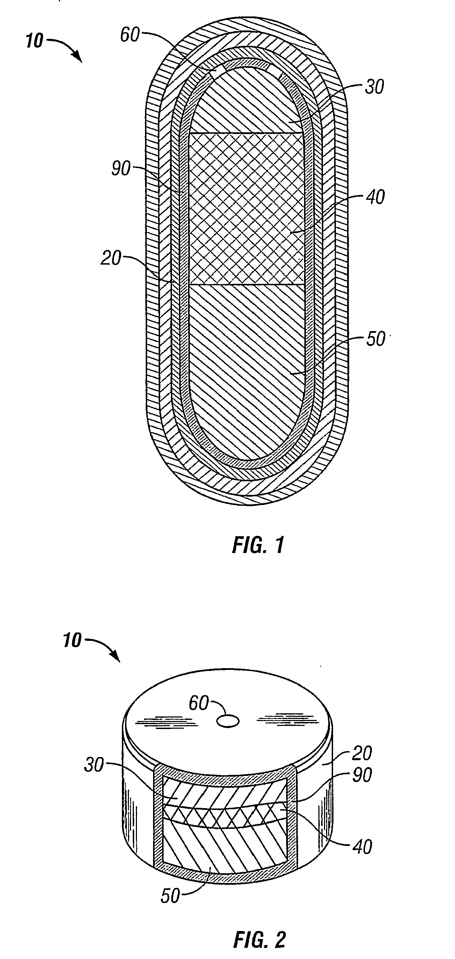

Figure 1 is a cutaway view of a capsule shaped tablet of dosage form 10. The internal compartment defined by membrane 20 contains a multilayer-compressed core having a first component non-drug delay layer 30, a second component drug layer 40 and a third component push layer 50.

Figure 2 illustrates an alternate standard biconvex shape tablet. While Figure 1 illustrates a capsule-shaped tablet, the tablet geometry may be other shapes including a standard biconvex shape as illustrated in Figure 2. However, alternate shapes will alter release rates.

In operation, following oral ingestion of dosage form 10, the osmotic activity gradient across wall 20 causes gastric fluid to be imbibed through wall 20 thereby converting delay layer 30 and drug layer 40 into deliverable compositions, i.e. solutions or suspensions, and concurrently swelling the osmopolymer(s) in push layer 50. The deliverable delay layer 30 and drug layer 40 are released sequentially through exit 60 as fluid continues to enter the internal compartment and push layer 50 continues to swell. As release of delay layer 30 and drug layer 40 occurs, fluid continues to be imbibed and push layer 50 continues to swell thereby driving continued release. In this manner, drug is released in a continuous and uniform manner over an extended time period after a predetermined delay time.

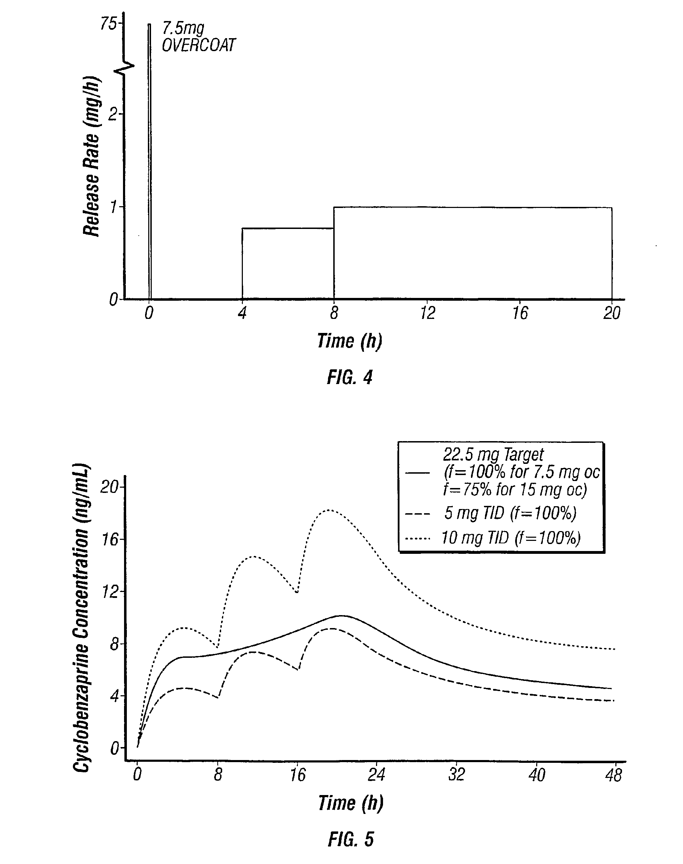

Figure 4 illustrates a model release rate profile for a 22.5 mg target system including an immediate release overcoat. The desired distinct demarcation of the beginning and ending points of the delay period and release periods are shown.

Figure 5 illustrates blood plasma concentration profiles including a model profile for a 22.5 mg target system having an immediate release overcoat. The desired uniform ascending concentration is shown.

As described in more detail below, third component push layer 50 comprises osmotically active component(s), but does not contain active drug. The osmotically active component(s) in push layer 50 typically comprise an osmagent and one or more osmopolymer(s) having relatively large molecular weights which exhibit swelling as fluid is imbibed such that significant release of these osmopolymers through exit 60 does not occur. Additional excipients such as binders, lubricants, antioxidants and colorants may also be included in push layer 50. The third component layer is referred to herein as an expandable or a push layer since, as fluid is imbibed, the osmopolymer(s) swell and push against the deliverable drug formulation of the second component drug layer to thereby facilitate release of the drug formulation from the dosage form.

Delay layer 30 comprises osmotically active components, but does not contain drug. The osmotically active component(s) in the first component delay layer typically comprise an osmagent and one or more osmopolymer(s) having relatively small molecular weights which exhibit swelling as fluid is imbibed such that release of these osmopolymers through exit 60 occurs similar to that of drug layer 40. Additional excipients such as binders, lubricants, antioxidants and colorants may also be included in delay layer 30. The first component layer is referred to herein as a delay layer since, as fluid is imbibed, the layer hydrates and is delivered without any active drug released from the core prior to release of drug layer 40, thereby creating a predetermined delay in release of drug layer 40 from the dosage form. Maintaining the viscosity of the hydrated delay layer greater than the viscosity of the hydrated drug layer throughout the delay period assists in assuring that minimal active drug is released from the core during the predetermined delay period.

Drug layer 40 comprises cyclobenzaprine in an admixture with selected excipients adapted to provide an osmotic activity gradient for driving fluid from an external environment through membrane 20 and for forming a deliverable drug formulation upon imbibition of fluid. The excipients may include a suitable suspending agent, also referred to herein as a drug carrier, and an osmotically active agent, i.e., an "osmagent." Other excipients such as lubricants, binders, etc. may also be included.

Drug layer 40 further comprises a hydrophilic polymer carrier. The hydrophilic polymer provides a particle in the drug composition that contributes to the controlled delivery of the active drug. Representative examples of these polymers are poly(alkylene oxide) of 100,000 to 750,000 number-average molecular weight, including poly(ethylene oxide), poly(methylene oxide), poly(butylene oxide) and poly(hexylene oxide); and a poly(carboxymethylcellulose) of 40,000 to 400,000 number-average molecular weight, represented by poly(alkali carboxymethylcellulose), poly(sodium carboxymethylcellulose), poly(potassium carboxymethylcellulose) and poly(lithium carboxymethylcellulose). Drug layer 40 can further comprise a hydroxypropylalkylcellulose of 9,200 to 125,000 number-average molecular weight for enhancing the delivery properties of the dosage form as represented by hydroxypropylethylcellulose, hydroxypropylmethylcellulose, hydroxypropylbutylcellulose and hydroxypropylpentylcellulose; and a poly(vinylpyrrolidone) of 7,000 to 75,000 number-average molecular weight for enhancing the flow properties of the dosage form. Preferred among these polymers are the poly(ethylene oxide) of 100,000 - 300,000 number average molecular weight. Carriers that erode in the gastric environment, i.e., bioerodible carriers, are especially preferred.

Other carriers that may be incorporated into drug layer 40 include carbohydrates that exhibit sufficient osmotic activity to be used alone or with other osmagents. Such carbohydrates comprise monosaccharides, disaccharides and polysaccharides. Representative examples include maltodextrins (i.e., glucose polymers produced by the hydrolysis of corn starch) and the sugars comprising lactose, glucose, raffinose, sucrose, mannitol, sorbitol, and the like. Preferred maltodextrins are those having a dextrose equivalence (DE) of 20 or less, preferably with a DE ranging from about 4 to about 20, and often 9-20. Maltodextrin having a DE of 9-12 has been found to be useful.

Drug layer 40 typically will be a substantially dry, <1% water by weight, composition formed by compression of the carrier, the drug, and other excipients as one layer.

Drug layer 40 may be formed from particles by comminution that produces the size of the drug and the size of the accompanying polymer used in the fabrication of the drug layer, typically as a core containing the compound, according to the mode and the manner of the invention. The means for producing particles include granulation, spray drying, sieving, lyophilization, crushing, grinding, jet milling, micronizing and chopping to produce the intended micron particle size. The process can be performed by size reduction equipment, such as a micropulverizer mill, a fluid energy grinding mill, a grinding mill, a roller mill, a hammer mill, an attrition mill, a chaser mill, a ball mill, a vibrating ball mill, an impact pulverizer mill, a centrifugal pulverizer, a coarse crusher and a fine crusher. The size of the particle can be ascertained by screening, including a grizzly screen, a flat screen, a vibrating screen, a revolving screen, a shaking screen, an oscillating screen and a reciprocating screen. The processes and equipment for preparing drug and carrier particles are disclosed in Pharmaceutical Sciences, Remington, 17th Ed., pp. 1585-1594 (1985); Chemical Engineers Handbook, Perry, 6th Ed., pp. 21-13 to 21-19 (1984); Journal of Pharmaceutical Sciences, Parrot, Vol. 61, No. 6, pp. 813-829 (1974); and Chemical Engineer, Hixon, pp. 94-103 (1990).

Optionally, surfactants and disintegrants may be utilized in the drug layer. Exemplary of the surfactants are those having an HLB value of between about 10 - 25, such as polyethylene glycol 400 monostearate, polyoxyethylene-4-sorbitan monolaurate, polyoxyethylene-20-sorbitan monooleate, polyoxyethylene-20-sorbitan monopalmitate, polyoxyethylene-20-monolaurate, polyoxyethylene-40 -stearate, sodium oleate and the like. Disintegrants may be selected from starches, clays, celluloses, algins and gums and crosslinked starches, celluloses and polymers. Representative disintegrants include corn starch, potato starch, croscarmelose, crospovidone, sodium starch glycolate, Veegum HV, methylcellulose, agar, bentonite, carboxymethylcellulose, alginic acid, guar gum and the like.

The active compound may be provided in the drug layer in amounts of from 1 mg to 100 mg per dosage form, preferably 10mg - 40mg per dosage form, depending upon the required dosing level that must be maintained over the delivery period, i.e., the time between consecutive administrations of the dosage forms. More typically, loading of compound in the dosage forms will provide doses of compound to the subject ranging from 10 mg to 60 mg per day, more usually 20 mg to 40 mg per day. Generally, if a total drug dose of more than 100 mg per day is required, multiple units of the dosage form may be administered at the same time to provide the required amount of drug.

As a representative compound of the compounds having muscle-relaxing activity described herein, immediate release cyclobenzaprine is typically administered at a starting dose of 10 mg, administered three times per day. The effective dose range has been determined to be generally 20 mg/day to 60 mg/day.

Blood plasma concentrations in a subject may be determined by clinical assay to determine a correlation between tolerability and clinical effect and blood plasma concentrations of drug. Plasma concentrations may range from 3 ng/ml to 100 ng/ml (nanograms per milliliter), more typically 4 ng/ml to 40 ng/ml, of compound. The present invention provides for a period of delivery utilizing a substantially ascending blood plasma concentration profile.

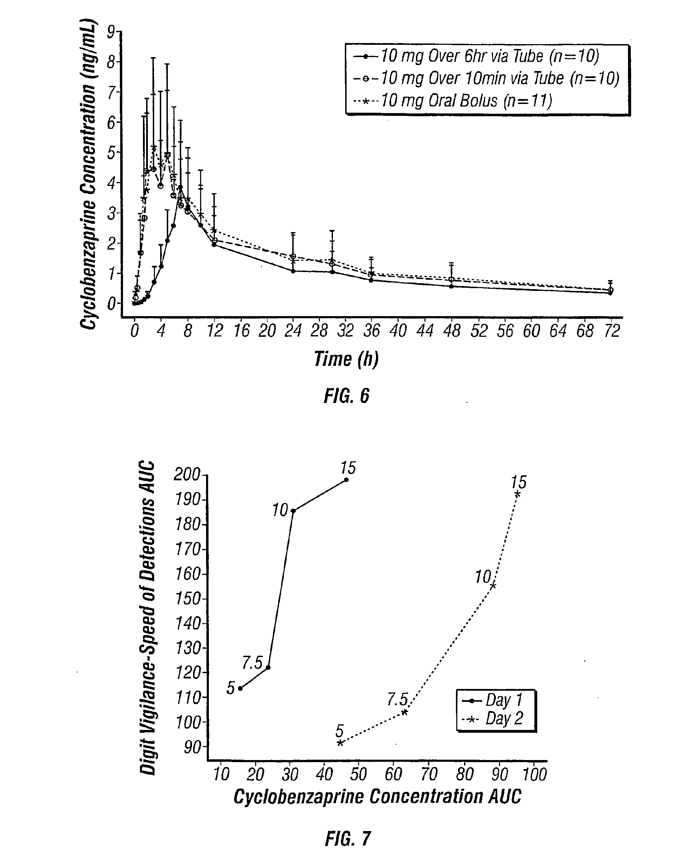

Figure 6 illustrates blood plasma concentrations at various times achieved through various means of delivery. This figure illustrates the time necessary to achieve a particular blood plasma concentration.

Figure 7 illustrates the affect of cyclobenzaprine on cognitive ability following various cyclobenzaprine treatments and the impairment associated with high concentrations of cyclobenzaprine.

In presently preferred embodiments of once-a-day dosage forms in accord with the present invention, the drug layer comprises cyclobenzaprine in a dose of 10 mg to 40 mg cyclobenzaprine per dosage form.

Dosage forms of the present invention have core drug release T₉₀ values of greater than 12 hours, preferably greater than 16 hours and most preferably greater than 18 hours, and released cyclobenzaprine for a continuous period of time of about 20. After about four hours following administration, the dosage form releases cyclobenzaprine from the core at a substantially ascending rate of release that continues for a prolonged period of time of about 16 hours or more. This release in the preferred embodiment occurred subsequent to release of the immediate release coating and the delay layer.

Wall 20 is formed to be permeable to the passage of an external fluid, such as water and biological fluids, and is substantially impermeable to the passage of cyclobenzaprine, osmagent, osmopolymer and the like. As such, it is semipermeable. The selectively semipermeable compositions used for forming wall 20 are essentially nonerodible and substantially insoluble in biological fluids during the life of the dosage form.

Representative polymers for forming wall 20 comprise semipermeable homopolymers, semipermeable copolymers, and the like. Such materials comprise cellulose esters, cellulose ethers and cellulose ester-ethers. The cellulosic polymers have a degree of substitution (DS) of their anhydroglucose unit of from greater than 0 up to 3, inclusive. Degree of substitution (DS) means the average number of hydroxyl groups originally present on the anhydroglucose unit that are replaced by a substituting group or converted into another group. The anhydroglucose unit can be partially or completely substituted with groups such as acyl, alkanoyl, alkenoyl, aroyl, alkyl, alkoxy, halogen, carboalkyl, alkylcarbamate, alkylcarbonate, alkylsulfonate, alkysulfamate, semipermeable polymer forming groups, and the like, wherein the organic moieties contain from one to twelve carbon atoms, and preferably from one to eight carbon atoms.

The semipermeable compositions typically include a member selected from the group consisting of cellulose acylate, cellulose diacylate, cellulose triacylate, cellulose acetate, cellulose diacetate, cellulose triacetate, mono-, di- and tri-cellulose alkanylates, mono-, di-, and tri-alkenylates, mono-, di-, and tri-aroylates, and the like. Exemplary polymers include cellulose acetate having a DS of 1.8 to 2.3 and an acetyl content of 32 to 39.9%; cellulose diacetate having a DS of 1 to 2 and an acetyl content of 21 to 35%; cellulose triacetate having a DS of 2 to 3 and an acetyl content of 34 to 44.8%; and the like. More specific cellulosic polymers include cellulose propionate having a DS of 1.8 and a propionyl content of 38.5%; cellulose acetate propionate having an acetyl content of 1.5 to 7% and an acetyl content of 39 to 42%; cellulose acetate propionate having an acetyl content of 2.5 to 3%, an average propionyl content of 39.2 to 45%, and a hydroxyl content of 2.8 to 5.4%; cellulose acetate butyrate having a DS of 1.8, an acetyl content of 13 to 15%, and a butyryl content of 34 to 39%; cellulose acetate butyrate having an acetyl content of 2 to 29%, a butyryl content of 17 to 53%, and a hydroxyl content of 0.5 to 4.7%; cellulose triacylates having a DS of 2.6 to 3, such as cellulose trivalerate, cellulose trilamate, cellulose tripalmitate, cellulose trioctanoate and cellulose tripropionate; cellulose diesters having a DS of 2.2 to 2.6, such as cellulose disuccinate, cellulose dipalmitate, cellulose dioctanoate, cellulose dicaprylate, and the like; and mixed cellulose esters, such as cellulose acetate valerate, cellulose acetate succinate, cellulose propionate succinate, cellulose acetate octanoate, cellulose valerate palmitate, cellulose acetate heptanoate, and the like. Semipermeable polymers are known in U.S. Patent No. 4,077,407, and they can be synthesized by procedures described in Encyclopedia of Polymer Science and Technology, Vol. 3, pp. 325-354 (1964), Interscience Publishers Inc., New York, NY.

Additional semipermeable polymers for forming wall 20 comprise cellulose acetaldehyde dimethyl acetate; cellulose acetate ethylcarbamate; cellulose acetate methyl carbamate; cellulose dimethylaminoacetate; semipermeable polyamide; semipermeable polyurethanes; semipermeable sulfonated polystyrenes; cross-linked selectively semipermeable polymers formed by the coprecipitation of an anion and a cation, as disclosed in U.S. Patents Nos. 3,173,876; 3,276,586; 3,541,005; 3,541,006 and 3,546,142; semipermeable polymers, as disclosed by Loeb, et al. in U.S. Patent No. 3,133,132; semipermeable polystyrene derivatives; semipermeable poly(sodium styrenesulfonate); semipermeable poly(vinylbenzyltrimethylammonium chloride); and semipermeable polymers exhibiting a fluid permeability of 10⁻⁵ to 10⁻² (cc. mil/cm hr.atm), expressed as per atmosphere of hydrostatic or osmotic pressure differences across a semipermeable wall. The polymers are known to the art in U.S. Patents Nos. 3,845,770; 3,916,899 and 4,160,020; and in Handbook of Common Polymers, Scott and Roff (1971) CRC Press, Cleveland, OH.

Wall 20 may also comprise a flux-regulating agent. The flux regulating agent is a compound added to assist in regulating the fluid permeability or flux through wall 20. The flux-regulating agent can be a flux-enhancing agent or a flux-decreasing agent. The agent can be preselected to increase or decrease the liquid flux. Agents that produce a marked increase in permeability to fluid such as water are often essentially hydrophilic, while those that produce a marked decrease to fluids such as water are essentially hydrophobic. The amount of regulator in the wall when incorporated therein generally is from about 0.01 % to 20% by weight or more. The flux regulator agents may include polyhydric alcohols, polyalkylene glycols, polyalkylenediols, polyesters of alkylene glycols, and the like. Typical flux enhancers include polyethylene glycol 300, 400, 600, 1500, 4000, 6000 and the like; low molecular weight glycols such as polypropylene glycol, polybutylene glycol and polyamylene glycol: the polyalkylenediols such as poly(1,3-propanediol), poly(1,4-butanediol), poly(1,6-hexanediol), and the like; aliphatic diols such as 1,3-butylene glycol, 1,4-pentamethylene glycol, 1,4-hexamethylene glycol, and the like; alkylene triols such as glycerine, 1,2,3-butanetriol, 1,2,4-hexanetriol, 1,3,6-hexanetriol and the like; esters such as ethylene glycol dipropionate, ethylene glycol butyrate, butylene glycol dipropionate, glycerol acetate esters, and the like. Presently preferred flux enhancers include the group of difunctional block-copolymer polyoxyalkylene derivatives of propylene glycol known as pluronics (BASF). Representative flux-decreasing agents include phthalates substituted with an alkyl or alkoxy or with both an alkyl and alkoxy group such as diethyl phthalate, dimethoxyethyl phthalate, dimethyl phthalate, and [di(2-ethylhexyl) phthalate], aryl phthalates such as triphenyl phthalate, and butyl benzyl phthalate; insoluble salts such as calcium sulfate, barium sulfate, calcium phosphate, and the like; insoluble oxides such as titanium oxide; polymers in powder, granule and like form such as polystyrene, polymethylmethacrylate, polycarbonate, and polysulfone; esters such as citric acid esters esterified with long chain alkyl groups; inert and substantially water impermeable fillers; resins compatible with cellulose based wall forming materials, and the like.

Other materials may be included in the semipermeable wall composition for imparting flexibility and elongation properties, for making wall 20 less brittle and to render tear strength. Suitable materials include phthalate plasticizers such as dibenzyl phthalate, dihexyl phthalate, butyl octyl phthalate, straight chain phthalates of six to eleven carbons, di-isononyl phthalte, diisodecyl phthalate, and the like. The plasticizers include nonphthalates such as triacetin, dioctyl azelate, epoxidized tallate, tri-isoctyl trimellitate, tri-isononyl trimellitate, sucrose acetate isobutyrate, epoxidized soybean oil, and the like. The amount of plasticizer in a wall when incorporated therein is about 0.01 % to 20% weight, or higher.

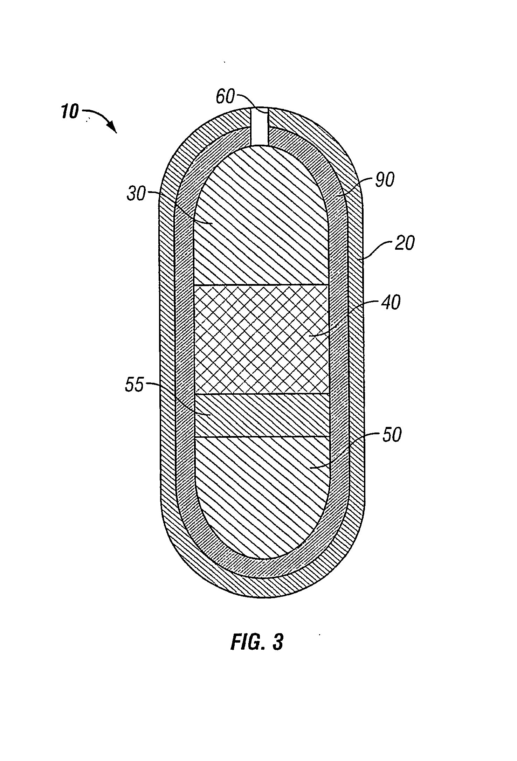

Push layer 50, the third component, comprises an expandable composition in contacting layered arrangement with the second component drug layer 40 as illustrated in Figure 1 or in contacting layered arrangement with barrier layer 55 as illustrated in Figure 3. Push layer 50 comprises a polymer that imbibes an aqueous or biological fluid and swells to push the drug composition through the exit means of the device. A polymer having suitable imbibition properties may be referred to herein as an osmopolymer. The osmopolymers are swellable, hydrophilic polymers that interact with water and aqueous biological fluids and swell or expand to a high degree, typically exhibiting a 2-50 fold volume increase. The osmopolymer can be non-crosslinked or crosslinked, but in a preferred embodiment are at least lightly crosslinked to create a polymer network that is too large and entangled to exit the dosage form. Thus, in a preferred embodiment, the expandable composition is retained within the dosage form during its operative lifetime.

Representatives of fluid-imbibing displacement polymers comprise members selected from poly(alkylene oxide) of 1 million to 15 million number-average molecular weight, as represented by poly(ethylene oxide), and poly(alkali carboxymethylcellulose) of 500,000 to 3,500,000 number-average molecular weight, wherein the alkali is sodium, potassium or lithium. Examples of additional polymers for the formulation of the push layer composition comprise osmopolymers that form hydrogels, such as Carbopol^{®} acidic carboxypolymer, a polymer of acrylic cross-linked with a polyallyl sucrose, also known as carboxypolymethylene, and carboxyvinyl polymer having a molecular weight of 250,000 to 4,000,000; Cyanamer^{®} polyacrylamides; cross-linked water swellable indenemaleic anhydride polymers; Good-rite^{®} polyacrylic acid having a molecular weight of 80,000 to 200,000; Aqua-Keeps^{®} acrylate polymer polysaccharides composed of condensed glucose units, such as diester cross-linked polygluran; and the like. Representative polymers that form hydrogels are known to the prior art in U.S. Patent No. 3,865,108, issued to Hartop; U.S. Patent No. 4,002,173, issued to Manning; U.S. Patent No. 4,207,893, issued to Michaels; and in Handbook of Common Polymers, Scott and Roff, Chemical Rubber Co., Cleveland, OH.

Suitable osmagents, also known as osmotic solutes and osmotically effective agents, that may be found in the drug layer, delay layer and the push layer in the dosage form are those which exhibit an osmotic activity gradient across the wall 20. Suitable osmagents comprise a member selected from the group consisting of sodium chloride, potassium chloride, lithium chloride, magnesium sulfate, magnesium chloride, potassium sulfate, sodium sulfate, lithium sulfate, potassium acid phosphate, mannitol, urea, inositol, magnesium succinate, tartaric acid, raffinose, sucrose, glucose, lactose, sorbitol, inorganic salts, organic salts and carbohydrates.

Exemplary solvents suitable for manufacturing the dosage form components comprise aqueous or inert organic solvents that do not adversely harm the materials used in the system. The solvents broadly include members selected from the group consisting of aqueous solvents, alcohols, ketones, esters, ethers, aliphatic hydrocarbons, halogenated solvents, cycloaliphatics, aromatics, heterocyclic solvents and mixtures thereof. Typical solvents include acetone, diacetone alcohol, methanol, ethanol, isopropyl alcohol, butyl alcohol, methyl acetate, ethyl acetate, isopropyl acetate, n-butyl acetate, methyl isobutyl ketone, methyl propyl ketone, n-hexane, n-heptane, ethylene glycol monoethyl ether, ethylene glycol monoethyl acetate, methylene dichloride, ethylene dichloride, propylene dichloride, carbon tetrachloride nitroethane, nitropropane tetrachloroethane, ethyl ether, isopropyl ether, cyclohexane, cyclooctane, benzene, toluene, naphtha, 1,4-dioxane, tetrahydrofuran, diglyme, water, aqueous solvents containing inorganic salts such as sodium chloride, calcium chloride, and the like, and mixtures thereof such as acetone and water, acetone and methanol, acetone and ethyl alcohol, methylene dichloride and methanol, and ethylene dichloride and methanol.

Figure 3 illustrates a trilayered-compressed core including an optional third component barrier layer 55 separating drug layer 40 from push layer 50. Figure 3 also illustrates dosage form 10 including inner wall 90.

The composition of barrier layer 55 is inert with the respect to the composition of the drug layer 40 and substantially impermeable; such that drug from drug layer 40 and the components of push layer 50 are prevented from mixing. Suitable materials include water-insoluble polymers, fats, fatty acids and fatty acid esters that are solids at ambient and body temperatures, and waxes. Representative water-insoluble polymers include ethyl cellulose, cellulose acetate, polyvinylchloride, copolymers of polyethylene and vinyl acetate, poly(methylmethacrylate), acrylic polymers such as Eudragit^{®} L or Eudragit^{®} R, polycaprolactone, poly(lactic-co-glycolic) acid polymers (PLGA), high density polyethylene, rubber, styrene butadiene, polysilicone, nylon, , polystyrene, polytetrafluoroethylene, and halogenated polymers. Representative waxes include paraffin wax and beeswax. Representative fats, fatty acids and fatty acid esters include C₁₆ - C₂₄ long chain fatty acids, esters of such long chain fatty acids such as stearic acid and oleic acid, and mixtures of the foregoing. Mixtures of the above-described materials may be utilized, e.g., a mixture of ethyl cellulose and stearic acid, which is presently preferred.

Inner wall 90 is permeable to the passage of gastric fluid entering the compartment defined by wall 20 and provides a lubricating function that facilitates the movement of delay layer 30, drug layer 40 and push layer 50 toward exit 60. Inner wall 90 may be formed from hydrophilic materials and excipients. Outer wall 20 is semipermeable, allowing gastric fluid to enter the compartment, but preventing the passage of the materials comprising the core in the compartment. The deliverable cyclobenzaprine formulation is released from exit 60 as described above with respect to the embodiment of Figure 3.

Inner wall 90 is located between at least the drug layer and the semipermeable wall to reduce friction between the external surface of delay layer 30 and drug layer 40, and the inner surface of wall 20. Inner wall 90 promotes release of the drug composition from the compartment and reduces the amount of residual drug composition remaining in the compartment at the end of the delivery period, particularly when the slurry, suspension or solution of the drug composition that is being dispensed is highly viscous during the period of time in which it is being dispensed. In dosage forms in which there is high drug loading, i.e., 40% or greater active agent in the drug layer based on the overall weight of the drug layer, and no inner wall, it has been observed that significant residual amounts of drug may remain in the device after the period of delivery has been completed. In some instances, amounts of 20% or greater may remain in the dosage form at the end of a twenty-four hour period when tested in a release rate assay.

Inner wall 90 is formed as an inner coat of a flow-promoting agent, i.e., an agent that lowers the frictional force between the outer wall 20 and the external surface of drug layer 40. Inner wall 90 apparently reduces the frictional forces between outer wall 20 and the outer surface of drug layer 40, and delay layer 30, thus allowing for more complete delivery of drug from the device. Particularly in the case of active compounds having a high cost, such an improvement presents substantial economic advantages since it is not necessary to load the drug layer with an excess of drug to insure that the minimal amount of drug required will be delivered. Inner wall 90 may be formed as a coating applied over the compressed core.

Inner wall 90 typically may be 0.01 to 5 mm thick, more typically 0.5 to 5mm thick, and it comprises a member selected from hydrogels, gelatin, low molecular weight polyethylene oxides, e.g., less than 100,000 MW, hydroxyalkylcelluloses, e.g., hydroxyethylcellulose, hydroxypropylcellulose, hydroxyisopropylcelluose, hydroxybutylcellulose and hydroxyphenylcellulose, and hydroxyalkyl alkylcelluloses, e.g., hydroxypropyl methylcellulose, and mixtures thereof. The hydroxyalkylcelluloses comprise polymers having a 9,500 to 1,250,000 number-average molecular weight. For example, hydroxypropyl celluloses having number average molecular weights of between 80,000 to 850,000 are useful. The inner wall may be prepared from conventional solutions or suspensions of the aforementioned materials in aqueous solvents or inert organic solvents. Prefered materials for the inner wall include hydroxypropyl cellulose, hydroxyethyl cellulose, hydroxypropyl methyl cellulose, povidone [poly(vinylpyrrolidone)], polyethylene glycol, and mixtures thereof. More prefered are mixtures of hydroxypropyl cellulose and povidone, prepared in organic solvents, particularly organic polar solvents such as lower alkanols having 1-8 carbon atoms, preferably ethanol, mixtures of hydroxyethyl cellolose and hydroxypropyl methyl cellulose prepared in aqueous solution, and mixtures of hydroxyethyl cellulose and polyethylene glycol prepared in aqueous solution. Most preferably, the inner wall comprises a mixture of hydroxypropyl cellulose and providone prepared in ethanol. Conveniently, the weight of the inner wall applied to the compressed core may be correlated with the thickness of the inner wall and residual drug remaining in a dosage form in a release rate assay such as described herein. As such, during manufacturing operations, the thickness of the inner wall may be controlled by controlling the weight of the inner wall taken up in the coating operation.

When inner wall 90 is formed as a subcoat, i.e., by coating onto the tabletted composite including one or all of the drug layer, delay layer and push layer, the inner wall can fill in surface irregularities formed on the core by the tabletting process. The resulting smooth external surface facilitates slippage between the coated composite core and the semipermeable wall during dispensing of the drug, resulting in a lower amount of residual drug composition remaining in the device at the end of the dosing period. When inner wall 90 is fabricated of a gel-forming material, contact with water in the environment of use facilitates formation of the gel or gel-like inner coat having a viscosity that may promote and enhance slippage between outer wall 20 and drug layer 40.

Pan coating may be conveniently used to provide the completed dosage form, except for the exit orifice. In the pan coating system, the wall-forming composition for the inner wall or the outer wall, as the case may be, is deposited by successive spraying of the appropriate wall composition onto the compressed single, bilayered or trilayered core comprising the drug layer for the single layer core; the drug layer and the push layer for the bilayered core; or the drug layer, barrier layer and push layer for the trilayered core, accompanied by tumbling in a rotating pan. A pan coater is used because of its availability at commercial scale. Other techniques can be used for coating the compressed core. Once coated, the wall is dried in a forced-air oven or in a temperature and humidity controlled oven to free the dosage form of solvent(s) used in the manufacturing. Drying conditions will be conventionally chosen on the basis of available equipment, ambient conditions, solvents, coatings, coating thickness, and the like.

Other coating techniques can also be employed. For example, the wall or walls of the dosage form may be formed in one technique using the air-suspension procedure. This procedure consists of suspending and tumbling the compressed single, bilayer or trilayer core in a current of air and the semipermeable wall forming composition, until the wall is applied to the core. The air-suspension procedure is well suited for independently forming the wall of the dosage form. The air-suspension procedure is described in U.S. Patent No. 2,799,241; in J. Am. Pharm. Assoc., Vol. 48, pp. 451-459 (1959); and, ibid., Vol. 49, pp. 82-84 (1960). The dosage form also can be coated with a Wurster^{®} air-suspension coater using, for example, methylene dichloride methanol as a cosolvent for the wall forming material. An Aeromatic^{®} air-suspension coater can be used employing a cosolvent.

Dosage forms in accord with the present invention are manufactured by standard techniques except that the preferred compression sequence is inverted over the traditional compression sequence to achieve the optimal layer interface geometry of convex compared to the traditional concave as discussed further, below. For example, the dosage form may be manufactured by the wet granulation technique. In the wet granulation technique, the drug and carrier are blended using an organic solvent, such as denatured anhydrous ethanol, as the granulation fluid. The remaining ingredients can be dissolved in a portion of the granulation fluid, such as the solvent described above, and this latter prepared wet blend is slowly added to the drug blend with continual mixing in the blender. The granulating fluid is added until a wet blend is produced, which wet mass blend is then forced through a predetermined screen onto oven trays. The blend is dried for 18 to 24 hours at 24°C to 35°C in a forced-air oven. The dried granules are then sized. Next, magnesium stearate, or another suitable lubricant, is added to the drug granulation, and the granulation is put into milling jars and mixed on a jar mill for 10 minutes. The composition is pressed into a layer, for example, in a Manesty^{®} press or a Korsch LCT press. For a bilayered core, the drug-containing layer is pressed and a similarly prepared wet blend of the push layer composition, if included, is pressed against the drug-containing layer. In the case of formation of the trilayered core, granules or powders of the drug layer composition, delay layer composition and push layer composition are sequentially placed in an appropriately-sized die with intermediate compression steps being applied to each of the first two layers, followed by a final compression step after the last layer is added to the die to form the trilayered core. The intermediate compression typically takes place under a force of about 50-100 newtons. Final stage compression typically takes place at a force of 3500 newtons or greater, often 3500-5000 newtons. The single, bilayer or trilayer compressed cores are fed to a dry coater press, e.g., Kilian^{®} Dry Coater press, and subsequently coated with the wall materials as described above. The compression sequence starts with the delay layer for a trilayer system followed by the drug layer and last, the push layer rather than the traditional sequence starting with the push layer. For a bilayer system, compression would begin with the drug layer followed by the push layer.

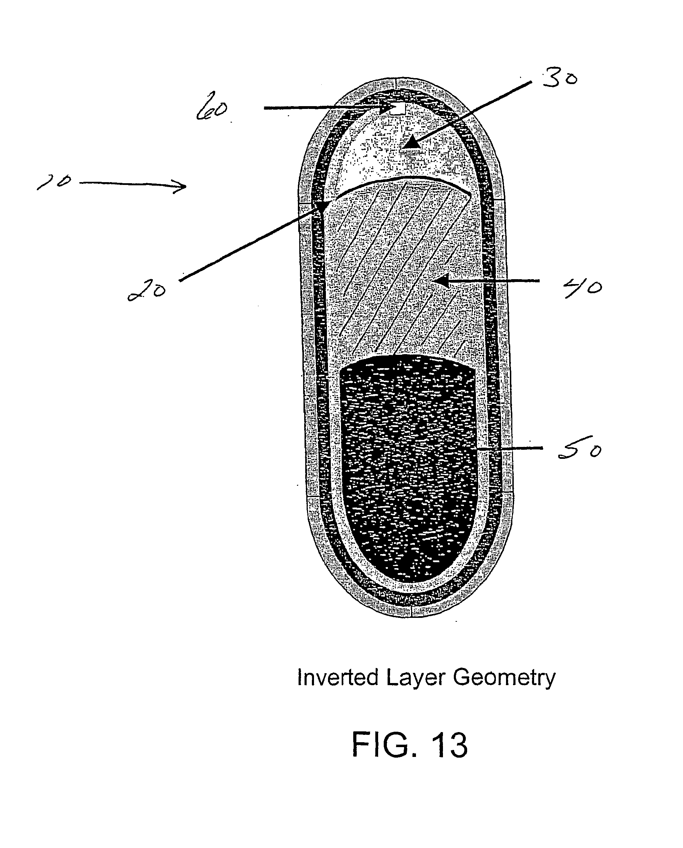

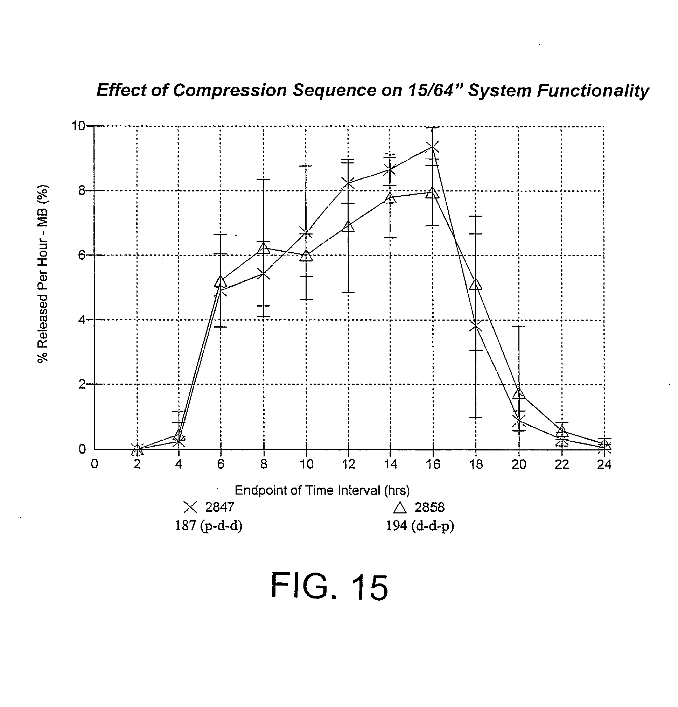

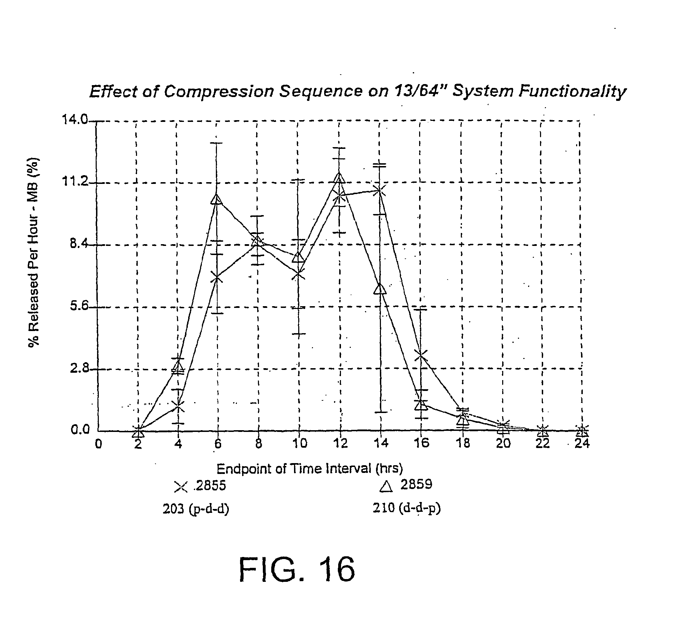

Figure 13 illustrates the resulting layer interfaces from the present invention. The traditional compression sequence is inverted, reversed, such that the natural shape of the layer interfaces from compression is inverted to be convex relative to the exit orifice for optimal performance. The result is that the perimeter of the layer interfaces is further separated from the delivery orifice at the outer perimeter of the core, adjacent to the semipermeable membrane. As a result, the localized flow, and subsequent mixing, condition set up by layer hydration at the core-membrane interface is not as significant in impacting the characteristic release profile since the boundary layer-orifice distance is greater. Typically, this reduced effect manifests itself in a more continuous and uniform ascending release profile as illustrated in Figure 15 and Figure 16.

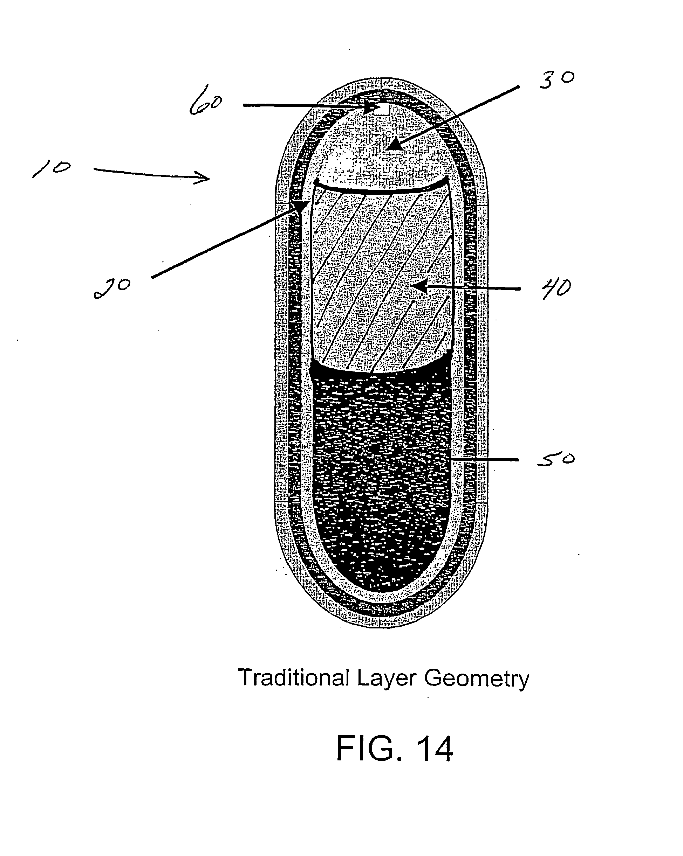

Figure 14 illustrates the resulting layer interfaces from the traditional compression sequence. Traditionally, for a multilayer tablet, the push layer composition, layer 50, is added to the press first and is compressed. Followed by, in this case for example, the drug layer 40, and finally by delay layer 30. In this manner, the traditional compression sequence results in the interfaces between the layers have a concave shape relative to the exit orifice as seen in Figure 14.

Figure 15 and Figure 16 illustrate the release rates from representative dosage forms that are identical except for their compression sequences. From these figures it can be seen that the inverted compression sequence of the present invention provides for an improved release profile that is more uniform.

One or more exit orifices are drilled in the drug layer end of the dosage form, and optional water soluble overcoats, which may be colored (e.g., Opadry colored coatings) or clear (e.g., Opadry Clear), may be coated on the dosage form to provide the finished dosage form.

In another manufacture the drug and other ingredients comprising the drug layer are blended and pressed into a solid layer. The layer possesses dimensions that correspond to the internal dimensions of the area the layer is to occupy in the dosage form, and it also possesses dimensions corresponding to the push layer, if included, for forming a contacting arrangement therewith. The drug and other ingredients can also be blended with a solvent and mixed into a solid or semisolid form by conventional methods, such as ballmilling, calendering, stirring or rollmilling, and then pressed into a preselected shape. Next, if included, a layer of osmopolymer composition is placed in contact with the layer of drug in a like manner. The layering of the drug formulation and the osmopolymer layer can be fabricated by conventional two-layer press techniques. An analogous procedure may be followed for the preparation of the trilayered core in which the delay layer is present. The compressed cores then may be coated with the inner wall material and the semipermeable wall material as described above.

Another manufacturing process that can be used comprises blending the powdered ingredients for each layer in a fluid bed granulator. After the powdered ingredients are dry blended in the granulator, a granulating fluid, for example, poly(vinylpyrrolidone) in water, is sprayed onto the powders. The coated powders are then dried in the granulator. This process granulates all the ingredients present therein while adding the granulating fluid. After the granules are dried, a lubricant, such as stearic acid or magnesium stearate, is mixed into the granulation using a blender e.g., V-blender or tote blender. The granules are then pressed in the manner described above.

The dosage form of the invention is provided with at least one exit 60. Exit 60 cooperates with the compressed core for the uniform release of drug from the dosage form. The exit can be provided during the manufacture of the dosage form or during drug delivery by the dosage form in a fluid environment of use.

Exit 60 may include an orifice that is formed or formable from a substance or polymer that erodes, dissolves or is leached from the outer wall to thereby form an exit orifice. The substance or polymer may include, for example, an erodible poly(glycolic) acid or poly(lactic) acid in the semipermeable wall; a gelatinous filament; a water-removable poly(vinyl alcohol); a leachable compound, such as a fluid removable pore-former selected from the group consisting of inorganic and organic salt, oxide and carbohydrate.

An exit, or a plurality of exits, can be formed by leaching a member selected from the group consisting of sorbitol, lactose, fructose, glucose, mannose, galactose, talose, sodium chloride, potassium chloride, sodium citrate and mannitol to provide a uniform-release dimensioned pore-exit orifice.

The exit can have any shape, such as round, triangular, square, elliptical and the like for the uniform metered dose release of a drug from the dosage form.

The dosage form can be constructed with one or more exits in spaced-apart relation or one or more surfaces of the dosage form.

Drilling, including mechanical and laser drilling, through the semipermeable wall can be used to form the exit orifice. Such exits and equipment for forming such exits are disclosed in U.S. Patents Nos. 3,916,899, by Theeuwes and Higuchi and in U.S. Patent No. 4,088,864, by Theeuwes, et al. It is presently preferred to utilize two exits of equal diameter.

Dosage forms of this invention exhibit sustained release of drug over a continuous time period that includes a prolonged time when drug is released at an ascending release rate as determined in a standard release rate assay such as that described herein. When administered to a subject, the dosage forms of the invention provide substantially ascending blood plasma drug concentrations in the subject that are less variable over a prolonged period of time than those obtained with immediate release dosage forms. When the dosage forms of this invention are administered on a continuous once-a-day basis, the dosage forms of the invention provide therapeutically effective ascending plasma cyclobenzaprine concentrations while providing steady-state peak plasma cyclobenzaprine concentrations that occur at a later time following dose administration and that exhibit a lesser magnitude than the steady-state peak plasma cyclobenzaprine concentrations that occur following twice or three times a day administration of an immediate-release cyclobenzaprine dosage form.

The practice of the foregoing methods of orally administering a cyclobenzaprine dosage form to a subject once a day is preferred. Other disease states and conditions, which may be manifested or diagnosed as muscle spasms, may be treated with the cyclobenzaprine dosage forms and methods of the invention. In addition, other disease states and conditions which may or may not manifest in association with depression but which may be responsive to treatment with cyclobenzaprine may also be treated with the dosage forms of the invention.

Preferred methods of manufacturing dosage forms described herein are generally described below. All percentages are weight percent unless otherwise noted.

Cyclobenzaprine systems with 0 mg overcoat and 14 mg core having core formulation utilizing Polyox^{®} of different viscosities (84 - 158 cps) and traditional compression sequence.

**Delay layer (56 mg)**

| | |
|---|---|
| 84.35% | Polyox^{®} WSR N-150 |
| 10.00% | NaCl |
| 5.00% | PVP K29-32 |
| 0.50% | Stearic Acid |
| 0.10% | Black Iron Oxide |
| 0.05% | BHT |

**Drug layer (93 mg)**

| | |
|---|---|
| 15.00% | Cyclobenzaprine HCI |
| 79.45% | Polyox^{®} WSR N-150 |
| 5.00% | PVP K29-32 |
| 0.50% | Stearic Acid |
| 0.05% | BHT |

**Push layer (168 mg)**

| | |
|---|---|
| 20.00% | Sodium Chloride |
| 73.70% | Polyethylene Oxide, NF, 7000K, TG |
| 5.00% | PVP K29-32 |
| 1.00% | Iron Oxide, Green PB-1581 |
| 0.25% | Stearic Acid |
| 0.05% | BHT |

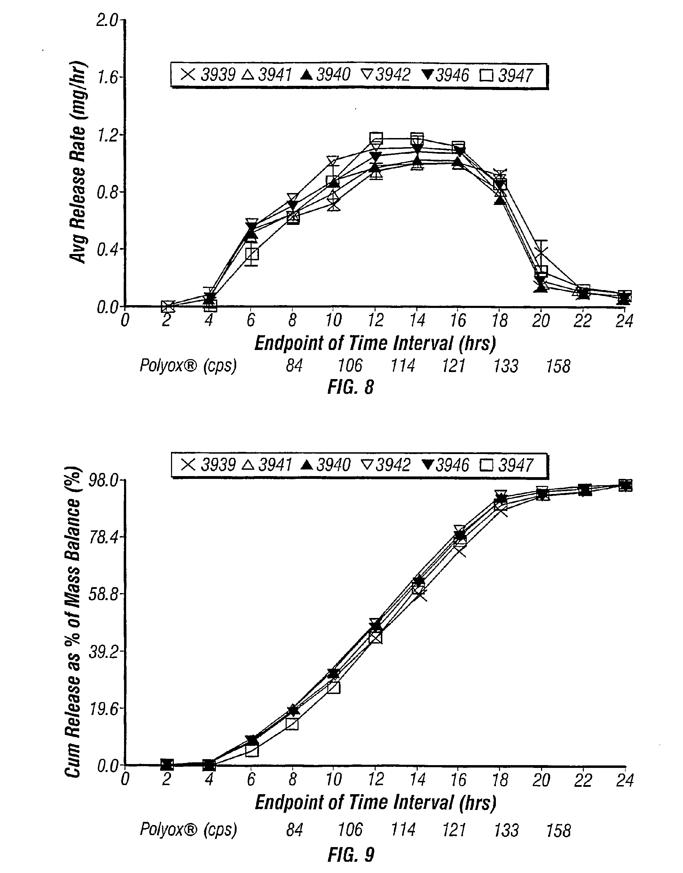

This formulation for dosage forms without an immediate release overcoat resulted in the release rate profiles and cumulative release rate profiles for the various polymer viscosities as depicted in Figure 8 and Figure 9.

### EXAMPLE 2

20 mg Cyclobenzaprine HCI systems with 6 mg overcoat and 14 mg core utilizing 109 cps viscosity core formulation

**Delay layer (56 mg)**

| | |
|---|---|
| 84.35% | Polyox^{®} WSR N-150 |
| 10.00% | NaCl |
| 5.00% | PVP K29-32 |
| 0.50% | Stearic Acid |
| 0.10% | Yellow Ferric Oxide |
| 0.05% | BHT |

**Drug layer (93 mg)**

| | |
|---|---|
| 15.00% | Cyclobenzaprine HCl |
| 79.45% | Polyox^{®} WSR N-150 |
| 5.00% | PVP K29-32 |
| 0.50% | Stearic Acid |
| 0.05% | BHT |

**Push layer (168 mg)**

| | |
|---|---|
| 20.00% | Sodium Chloride |
| 73.70% | Polyethylene Oxide, NF, 7000K, TG |
| 5.00% | PVP K29-32 |
| 1.00% | Iron Oxide, Green PB-1581 |
| 0.25% | Stearic Acid |
| 0.05% | BHT |

Formulation was modified prior to compression on the Korsch Multilayer press using 7/32" LCT, Deep Concave tooling. The individual layers were reduced to 168 mg (Push layer), 93 mg (Drug layer) and 56 mg (Delay layer).

Prior to wet granulation, the salt (NaCl) and ferric oxide were sieved using a 20-mesh screen (by hand, except for the push granulation where the milling was performed in the Quadro Comil equipped with a 21 mesh screen). For all the granulations, 50% of the povidone was sprayed as an aqueous solution (13% wt/wt) and the remaining 50% was charged as a powder to the batch. The drug layer was granulated using 10% excess drug to compensate for losses during the granulation process. Subsequent to the granulation, the dried mass was passed through a #7 mesh screen in the Granumill. The milled mass was then blended with butylated hydroxytoluene (BHT) for 10 min and stearic acid for 3 min in the GEMCO blender.

Compression was performed on the Multilayer Korsch press in the traditional compression sequence. The trilayer cores were membrane coated in the 24" Hi-Coater. Upon coating, cores were drilled to an orifice diameter of 45 mil using the LCT Laser. Each orifice was centered on the delay layer dome. Finally, drilled systems were dried at 45°C/ 45% relative humidity in a Hotpack^{™} oven for 84 hour followed by 3 hour of drying at 45°C/ambient humidity to reduce residual acetone levels from coating. Dried systems were subsequently drug, color and clear coated in the 24" Aqueous Coater.

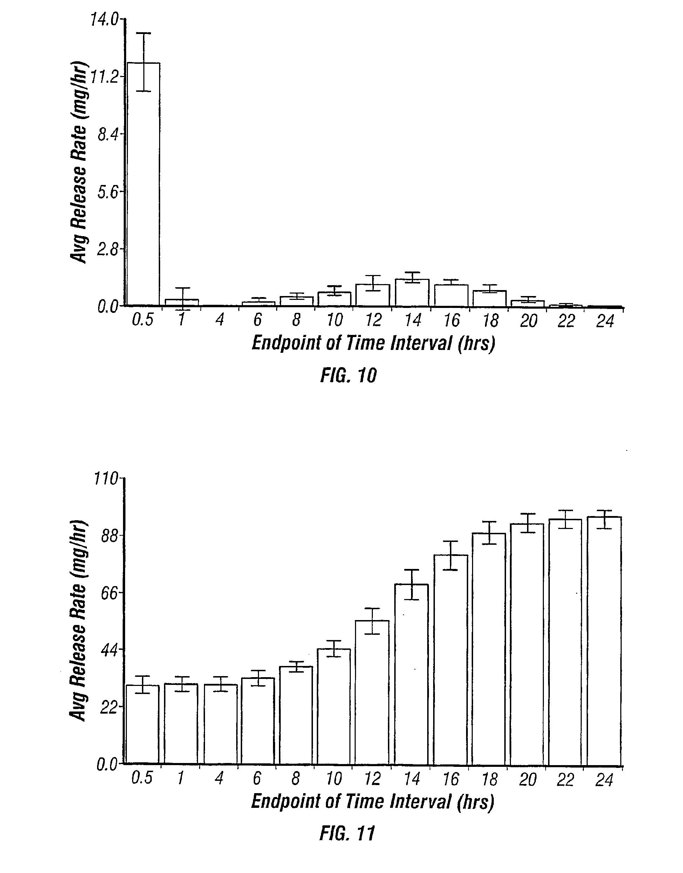

This formulation for a dosage form with an immediate release overcoat resulted in the release rate profiles and cumulative release rate profiles as depicted in Figure 10 and Figure 11.

## Claims

1. A dosage form comprising
(a) a membrane defining a compartment, the membrane having an exit orifice formed or formable therein and at least a portion of the membrane being semipermeable;
(b) an expandable layer located within the compartment remote from the exit orifice and in fluid communication with the semipermeable portion of the membrane;
(c) a delay layer located adjacent the exit orifice;
(d) a drug layer located within the compartment between the delay layer and the expandable layer; and
(e) an interface boundary between the delay layer and the drug layer, the interface boundary being convex in shape relative to the exit orifice.

2. The dosage form of claim 1, wherein the delay layer and the drug layer are formed by a compression sequence in which the delay layer is compressed into its form prior to the drug layer being compressed into its form.

3. The dosage form of claim 1 or 2 wherein:
the delay layer exhibits a higher viscosity than the viscosity of the drug layer when both are subjected to the same level of hydration.

4. The dosage form according to claim 3, wherein the delay layer and the drug layer each comprise a component present in the largest amount by weight, where the largest component by weight of the delay layer has a viscosity, when subjected to an aqueous medium, greater than the viscosity of the largest component by weight of the drug layer in the same aqueous medium at the same level of hydration.

5. The dosage form according to any preceding claim, wherein the delay layer provides a delay period, and after the delay period, drug from the drug layer is released from the dosage form for a period of time in a continuous and substantially ascending rate.

6. The dosage form according to any one of claims 3 to 5 wherein the viscosities of the drug layer and the delay layer during operation are between 70 and 350 cps (mPa·s)

7. The dosage form of claim 3 wherein the viscosity of the delay layer is above 150 cps (mPa·s) in an aqueous medium.

8. The dosage form of any preceding claim wherein the drug layer comprises a drug selected from the group of cyclobenzaprine, amitriptyline, imipramine and desipramine.

9. The dosage form of any preceding claim wherein the drug layer comprises cyclobenzaprine and that provides a cyclobenzaprine plasma concentration of 6 to 8 ng/ml three to four hours after dosing and approximately 8 to 1 2 ng/ml eighteen to twenty hours after oral administration in a human.

10. The dosage form of any one of claims 1 to 7, wherein the drug layer comprises a tricyclic amine.

11. A dosage form according to any one of claims 1 to 10, for use in reducing tunneling of a drug layer through a delay layer of a delayed release dosage form during a delay period, wherein the delay layer and the drug layer are formulated such that the viscosity of the delay layer remains higher than the viscosity of the drug layer during the delay period.

12. A dosage form according to any one of claims 1 to 10, for use in controlling the release of a drug layer from a delayed release dosage form, wherein the delay layer and the drug layer are formulated such that the viscosity of hydrated portions of the delay layer within the compartment remains higher than the viscosity of the hydrated portions of the drug layer within the compartment during a substantial portion of the time that the delay layer inhibits the release of the drug layer from the compartment.

## Patentansprüche

1. Dosierungsform umfassend
(a) eine Membran, die ein Kompartment definiert, die Membran hat eine Auslaßöffnung, die darin geformt wird oder formbar ist und wenigstens ein Teil der Membran ist semipermeabel;
(b) eine expandierbare Schicht, die innerhalb des Kompartiments lokalisiert ist, die von der Auslaßöffnung entfernt ist und in flüssiger Kommunikation mit dem semipermeablen Teil der Membran ist;
(c) eine Verzögerungsschicht, die benachbart der Auslaßöffnung lokalisiert ist;
(d) eine Arzneimittelschicht, die innerhalb des Kompartiments zwischen der Verzögerungsschicht und der expandierbaren Schicht lokalisiert ist; und
(e) eine Oberflächenbegrenzung zwischen der Verzögerungsschicht und der Arzneimittelschicht, die Übergangsflächenabgrenzung hat in ihrer Ausformung hinsichtlich der Auslaßöffnung eine konvexe Form.

2. Dosierungsform nach Anspruch 1, bei der die Verzögerungsschicht und die Arzneimittelschicht durch eine Kompressionssequenz gebildet sind, bei der die Verzögerungsschicht in ihre Form gepreßt wird, bevor die Arzneimittelschicht in ihre Form gepreßt wird.

3. Dosierungsform nach Anspruch 1 oder 2, bei der
die Verzögerungsschicht eine höhere Viskosität als die Viskosität der Arzneimittelschicht aufweist, wenn beide dem gleichen Level an Hydratation unterzogen werden.

4. Dosierungsform nach Anspruch 3, bei der die Verzögerungsschicht und die Arzneimittelschicht jeweils eine Komponente umfassen, die in der nach Gewicht größten Menge vorhanden ist, wobei die nach Gewicht größte Komponente der Verzögerungsschicht eine Viskosität hat, wenn sie einem wässerigen Medium ausgesetzt wird, die größer als die Viskosität der nach Gewicht größten Komponente der Arzneimittelschicht in dem gleichen wässerigen Medium beim gleichen Level der Hydratation ist.

5. Dosierungsform nach einem der vorherigen Ansprüche, bei der die Verzögerungsschicht für einen Verzögerungszeitraum sorgt und nach dem Verzögerungszeitraum Arzneimittel aus der Arzneimittelschicht aus der Dosierungsform über einen Zeitraum in einer kontinuierlichen und im wesentlichen ansteigenden Rate freigesetzt wird.

6. Dosierungsform nach einem der Ansprüche 3 bis 5, bei der die Viskositäten der Arzneimittelschicht und Verzögerungsschicht während der Durchführung zwischen 70 und 350 cps (mPa.s) liegen.

7. Dosierungsform nach Anspruch 3, bei der die Viskosität der Verzögerungsschicht über 150 cps (mPa·s) in einem wässerigen Medium ist.

8. Dosierungsform nach einem der vorherigen Ansprüche, bei der die Arzneimittelschicht ein Arzneimittel ausgewählt aus der Gruppe Cyclobenzaprin, Amitriptylin, Imipramin und Desipramin umfaßt.

9. Dosierungsform nach einem der vorherigen Ansprüche, bei der die Arzneimittelschicht Cyclobenzaprin umfaßt und die eine Cyclobenzaprin-Plasmakonzentration von 6 bis 8 ng/ml drei bis vier Stunden nach der Dosierung und von ungefähr 8 bis 12 ng/ml achtzehn bis zwanzig Stunden nach oraler Verabreichung bei einem Menschen bereitstellt.

10. Dosierungsform nach einem der Ansprüche 1 bis 7, bei der Arzneimittelschicht tricyclische Amine umfaßt.

11. Dosierungsform nach einem der Ansprüche 1 bis 10, die zur Reduzierung des Tunneleffekts einer Arzneimittelschicht durch eine Verzögerungsschicht einer verzögert freisetzenden Dosierungsform während eines Verzögerungszeitraums verwendet wird, wobei die Verzögerungsschicht und die Arzneimittelsähicht so formuliert sind, daß die Viskosität der Verzögerungsschicht höher als die Viskosität der Arzneimittelschicht während des Verzögerungszeitraums bleibt.

12. Dosierungsform nach einem der Ansprüche 1 bis 10, die zur Kontrolle der Freisetzung einer Arzneimittelschicht aus einer verzögert freisetzenden Dosierungsform verwendet wird, wobei die Verzögerungsschicht und die Arzneimittelschicht so formuliert sind, daß die Viskosität der hydratisierten Teile der Verzögerungsschicht innerhalb des Kompartiments höher als die Viskosität der hydratisierten Teile der Arzneimittelschicht innerhalb des Kompartiments während eines wesentlichen Anteils des Zeitraums aufrechterhalten wird, daß die Verzögerungsschicht die Freisetzung der Arzneimittelschicht aus dem Kompartiment inhibiert.

## Revendications

1. Forme posologique comprenant :
(a) une membrane définissant un compartiment, la membrane ayant un orifice de sortie formé ou apte à être formé dans celle-ci et au moins une partie de la membrane étant semiperméable ;
(b) une couche expansible située à l'intérieur du compartiment de façon éloignée de l'orifice de sortie et en communication de fluide avec la partie semiperméable de la membrane ;
(c) une couche retard située adjacente à l'orifice de sortie ;
(d) une couche de médicament située à l'intérieur du compartiment entre la couche retard et la couche expansible ; et
(e) une limite d'interface entre la couche retard et la couche de médicament, la limite d'interface étant de forme convexe par rapport à l'orifice de sortie.

2. Forme posologique selon la revendication 1, dans laquelle la couche retard et la couche de médicament sont formées par une séquence de compressions dans laquelle la couche retard est comprimée en sa forme avant que la couche de médicament ne soit comprimée en sa forme.

3. Forme posologique selon l'une des revendications 1 ou 2, dans laquelle :
- la couche retard présente une viscosité supérieure à la viscosité de la couche de médicament lorsque les deux sont soumises au même niveau d'hydratation.

4. Forme posologique selon la revendication 3,
dans laquelle la couche retard et la couche de médicament comprennent chacune un composant présent dans la quantité la plus importante en poids, où le composant le plus important en poids de la couche retard a une viscosité, lorsqu'il est soumis à un milieu aqueux, supérieure à la viscosité du composant le plus important en poids de la couche de médicament dans le même milieu aqueux au même niveau d'hydratation.

5. Forme posologique selon l'une quelconque des revendications précédentes, dans laquelle la couche retard assure une période de retard, et, après la période de retard, du médicament provenant de la couche de médicament est libéré de la forme posologique pendant une période de temps à une allure continue et sensiblement ascendante.

6. Forme posologique selon l'une quelconque des revendications 3 à 5, dans laquelle les viscosités de la couche de médicament et de la couche retard pendant le fonctionnement sont entre 70 et 350 cps (mPa.s).

7. - Forme posologique selon la revendication 3,
dans laquelle la viscosité de la couche retard est au-dessus de 150 cps (mPa.s) dans un milieu aqueux.

8. Forme posologique selon l'une quelconque des revendications précédentes, dans laquelle la couche de médicament comprend un médicament choisi dans le groupe de la cyclobenzaprine, l'amitriptyline, l'imipramine et la désipramine.

9. Forme posologique selon l'une quelconque des revendications précédentes, dans laquelle la couche de médicament comprend de la cyclobenzaprine et qui assure une concentration de cyclobenzaprine dans le plasma de 6 à 8 ng/ml trois à quatre heures après l'administration dosée et approximativement 8 à 12 ng/ml dix-huit à vingt heures après l'administration orale dans un être humain.

10. Forme posologique selon l'une quelconque des revendications 1 à 7, dans laquelle la couche de médicament comprend une amine tricyclique.

11. Forme posologique selon l'une quelconque des revendications 1 à 10, destinée à être utilisée dans la réduction de la tunnellisation d'une couche de médicament à travers une couche retard d'une forme posologique à libération retardée pendant une période de retard, la couche retard et la couche de médicament étant formulées de telle sorte que la viscosité de la couche retard reste supérieure à la viscosité de la couche de médicament pendant la période de retard.

12. Forme posologique selon l'une quelconque des revendications 1 à 10, destinée à être utilisée dans le contrôle de la libération d'une couche de médicament à partir d'une forme posologique à libération retardée, la couche retard et la couche de médicament étant formulées de telle sorte que la viscosité de parties hydratées de la couche retard à l'intérieur du compartiment reste supérieure à la viscosité des parties hydratées de la couche de médicament à l'intérieur du compartiment pendant une partie substantielle du temps où la couche retard inhibe la libération de la couche de médicament à partir du compartiment.
